# FASCICULE DE BREVET EUROPEEN

(11) **EP 1 819 655 B1**
(45) Date de publication et mention de la délivrance du brevet: **17.11.2010**
(21) Numéro de dépôt: 05822993.1
(22) Date de dépôt: 30.11.2005
(51) Int. Cl.: C07C 45/53, C07C 45/39, C07C 49/403, C07D 201/06

(54) **PROCEDE DE PREPARATION DE CYCLOHEXANONE**
VERFAHREN ZUR HERSTELLUNG VON CYCLOHEXANON
METHOD FOR THE PREPARATION OF CYCLOHEXANONE

(30) Priorité: 07.12.2004 FR 0412976
(43) Date de publication de la demande: 22.08.2007
(73) Titulaire: RHODIA CHIMIE, 93300 Aubervilliers (FR)
(72) Inventeur: LECONTE, Philippe, 68150 Ribeauvillé (FR); VERACINI, Serge, F-69005 Lyon (FR); MOREL, Philippe, F-38200 Chuzelles (FR)
(74) Mandataire: Esson, Jean-Pierre
(86) Numéro de dépôt international: PCT/FR2005/002979
(87) Numéro de publication internationale: WO 2006/061487

(56) Documents cités:
- EP-A- 0 450 498
- GB-A- 1 018 557
- US-A- 4 918 239

## Description

La présente invention concerne un procédé de préparation de cyclohexanone à partir de cyclohexane.

Elle concerne plus particulièrement un procédé de préparation de cyclohexanone présentant une teneur en impuretés permettant une utilisation de la cyclohexanone comme matière première pour la fabrication d'ε-caprolactame.

En effet, le polyamide 6 ou polycaprolactame est un des matériaux thermoplastiques majeurs pour la fabrication de fils, fibres ainsi que des pièces moulées diverses. Ce polymère est obtenu par polymérisation de l'ε-caprotactame.

Ce composé peut être obtenu par différents procédés. Parmi ceux-ci, l'un des plus utilisés consiste à produire la cyclohexanone oxime à partir de cyclohexanone.

Dans ce procédé, la cyclohexanone doit avoir un degré de pureté élevé pour éviter d'introduire des impuretés qui pourraient être gênantes notamment au cours de l'étape de polymérisation du caprolactame et qui pourraient également altérer les propriétés du polyamide obtenu, notamment la coloration du polyamide et sa résistance au vieillissement

La cyclohexanone est généralement obtenue par oxydation du cyclohexane à l'état liquide par un gaz contenant de l'oxygène pour donner un mélange cyclohexanol/cyclohexanone puis après purification et séparation du cyclohexanol, de la cyclohexanone, déshydrogénation du cyclohexanol en cyclohexanone.

Cette oxydation du cyclohexane en cyclohexanone/cyclohexanol est soit réalisée en une seule étape, en présence de catalyseur d'oxydation, soit dans une première étape d'oxydation du cyclohexane en hydroperoxyde de cyclohexyle, sans catalyseur, puis décomposition catalytique de cet hydroperoxyde en cyclohexanol et cyclohexanone.

Au cours de ces étapes d'oxydation, de nombreuses impuretés sont produites tels que des aldéhydes, acides, alcools et cétones. Ces impuretés ne peuvent généralement pas être transformées par la suite en produits valorisables tels que acide adipique ou ε-caprolactame. Ces impuretés doivent donc être éliminées et séparées de la cyclohexanone et/ou du cyclohexanol notamment dans le cas de la production de cyclohexanone.

Dans les procédés d'oxydation du cyclohexane en présence d'un catalyseur, ceux-ci comprennent une étape de purification du mélange Cyclohexanone/Cyclohexanol par un traitement basique ou une distillation en milieu basique. Des procédés de traitement du mélange cyclohexanol/cyclohexanone par des composés alcalins sont notamment décrits dans les documents JP5271143 et GB 1018557 A. Avec une tel traitement basique, les impuretés sont éliminées.

Toutefois, un tel procédé demande un traitement complémentaire avec utilisation d'un nouveau réactif, un composé basique tel qu'un hydroxyde métallique. Il est donc obligatoire de prévoir également une séparation et récupération de ce composé basique sous forme d'effluents qu'il est généralement nécessaire de traiter (incinération ou autre).
La demande de brevet EP 0450498 A décrit un procédé pour purifier les impuretés résiduelles qui n'ont pu être séparées par les procédés décrits ci-dessus_{.}

Dans le cas d'une oxydation du cyclohexane en hydroperoxyde de cyclohexyle, il est également possible de mettre en oeuvre un traitement par un composé basique, avec les mêmes avantages et inconvénients que ceux décrits ci-dessus.

Un des buts de la présente invention est de remédier à ces inconvénients en proposant un procédé de préparation de cyclohexanone à partir de l'oxydation du cyclohexane sans mettre en oeuvre un traitement de purification utilisant un composé basique.

A cet effet, l'invention propose un procédé de fabrication de cyclohexanone caractérisé en ce qu'il comprend les étapes suivantes :
- Oxyder du cyclohexane en hydroperoxyde de cyclohexyle par de l'oxygène ou un gaz contenant de l'oxygène en absence de catalyseur,
- purifier le milieu réactionnel par lavage à l'eau,
- Décomposer l'hydroperoxyde de cyclohexyle en cyclohexanol et cyclohexanone en présence d'un catalyseur,
- Récupérer le mélange cyclohexanol/cyclohexanone par séparation du cyclohexane n'ayant pas réagi et séparation des produits de point d'ébullition plus élevés que ceux du cyclohexanol/cyclohexanone
- Déshydrogéner le cyclohexanol contenu dans le mélange cyclohexanol/cyclohexanone en présence d'un catalyseur de déshydrogénation
- Distiller, dans une première étape de distillation, le mélange obtenu pour obtenir une fraction de tête (F₁) comprenant les composés de point d'ébullition inférieur à la température d'ébullition de la cyclohexanone, et une fraction de queue (Q₁)
- Distiller, dans une seconde étape de distillation, la fraction de queue (Q₁) pour obtenir une fraction de tête (F₂) constitué par la cyclohexanone, et une fraction de queue (Q₂).

La préparation du mélange cyclohexanol / cyclohexanone telle que décrite dans les premières étapes ne permet pas d'éliminer toutes les impuretés gênantes, et notamment l'α,β-cyclopentène-1 carboxaldéhyde (cyclopenténal) qu'on retrouve ainsi dans le mélange cyclohexanol / cyclohexanone.
Ce composé qui présente un point d'ébullition voisin de celui de la cyclohexanone est très difficile à séparer de celle-ci par distillation. Or, ce composé répond à certains tests de qualification couramment pratiqués pour qualifier la cyclohexanone pour notamment la synthèse du caprolactame, comme le test UV réalisé avec un rayon lumineux à une certaine longueur d'onde ou un test d'oxydation.
Dans le procédé de l'invention, ce composé, le cyclopenténal, est chimiquement transformé pendant l'étape de déshydrogénation en produits séparables de la cyclohexanone par, par exemple, une opération classique de distillation telle que celle prévue dans l'enchaînement d'opérations décrit plus haut.

L'étape de déshydrogénation du cyclohexanol contenu dans le mélange cyclohexanol/cyclohexanone est réalisée en présence d'un catalyseur de déshydrogénation et à des conditions de température et pression usuelles et décrites dans la littérature telles que, par exemple, une température comprise entre 200°C et 450°C et une pression absolue comprise entre 1 et 3 bars. A titre d'exemple de description de cette étape de déshydrogénation, on peut citer le brevet US4918239.

Dans un mode de réalisation de l'invention cette étape de déshydrogénation est mise en oeuvre en présence d'un catalyseur à base d'oxydes de cuivre, de magnésium, de zinc et/ou leurs mélanges.

Ainsi, le procédé de l'invention permet de produire une cyclohexanone répondant aux critères de pureté requis notamment pour la fabrication de l'ε-caprolactame, en particulier au test UV consistant à déterminer la transmission d'un rayon lumineux de longueur d'onde 230 nm- à travers un volume de cyclohexanone. Cette transmission doit être supérieure à 86 %:
Selon une autre caractéristique de l'invention, la fraction queue Q₂ est distillée dans une troisième étape de distillation pour obtenir une fraction de tête (F₃) constituée de cyclohexanol/cyclohexanone et une fraction de queue (Q₃) constituée par des composés de point d'ébullition élevés.

La fraction de tête (F₃) est avantageusement recyclée dans le flux de mélange cyclohexanol/cyclohexanone introduit dans l'étape de déshydrogénation du cyclohexanol.

Selon une autre caractéristique du procédé de l'invention, la fraction de tête (F₁) est distillée pour obtenir une nouvelle fraction de tête (F₄) constituée par des composés de point d'ébullition peu élevé, et une nouvelle fraction de queue (Q₄) constituée essentiellement de cyclohexanone. Cette fraction de queue (Q₄) est, avantageusement, recyclé dans le mélange cyclohexanol/cyclohexanone introduit à l'étape d'hydrogénation.

Le procédé de l'invention permet de récupérer une cyclohexanone, en fraction de tête (F₂), présentant des critères de pureté élevés et notamment un test UV (% transmission à = 230 nm supérieure à la spécification demandée). La cyclohexanone produite par le procédé de l'invention est donc, avantageusement utilisée pour la fabrication de l'ε caprolactame par oximation.

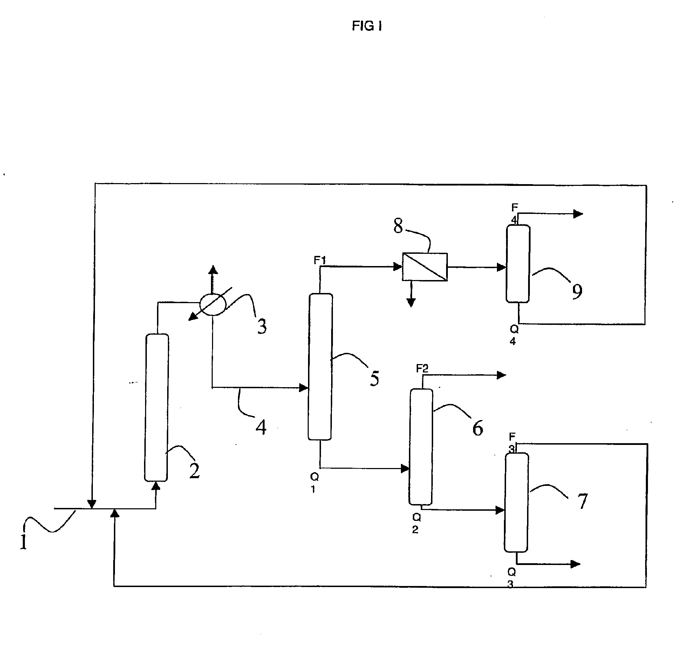

D'autres avantages, détails de l'invention apparaîtront plus clairement au vu des exemples donnés ci-dessous uniquement à titre indicatif et à la description d'un mode de réalisation du procédé de l'invention faite en référence à la figure unique annexée qui représente un schéma synoptique de ce mode de réalisation.

### Exemple 1:

Un mélange de cyclohexanol contenant 600 ppm de cyclopenténal est alimenté en 1 dans un réacteur colonne 2.
Dans la colonne 2 est disposé un catalyseur en lit fixe. Le catalyseur est à base d'oxyde de cuivre. La température dans la colonne 2 est de 230°C. Le taux de transformation du cyclohexanol en cyclohexanone est de 30%. La concentration en cyclopenténal dans le milieu réactionnel en sortie du réacteur 2 est inférieure à la limite détectable par les méthodes de mesure connues, à savoir, inférieure à 30 ppm.

### Exemple 2 :

On alimente dans le réacteur 2 un mélange contenant 59 % en poids de cyclohexanone, 39 % en poids de cyclohexanol, 0,5 % en poids d'eau et 1,5 % en poids de produits lourds ou légers considérés comme des impuretés à éliminer. Comme impuretés particulières on peut citer le cyclopenténal qui est présent à une concentration de 2950 ppm.

Le débit d'alimentation de ce mélange dans le réacteur 2 est de 215 g/h.

La température du réacteur est de 310°C.
Le milieu réactionnel en sortie de réacteur contient 80,6 % en poids de cyclohexanone, 16,5 % en poids de cyclohexanol et des impuretés lourdes ou légères. La concentration en cyclopenténal dans ce milieu est inférieure au seuil de détection, c'est à dire inférieure à 30 ppm.
Le taux de transformation du cyclohexanol en cyclohexanone est de 55%.

Le milieu réactionnel sortant du réacteur 2 est alimenté dans un échangeur de chaleur 3, puis par le conduit 4 dans une première colonne de distillation 5.
Cette colonne comprend 22 étages théoriques et fonctionnent sous des conditions de température et pression- habituelles et connnues de l'homme du métier dans le domaine de distillation de la cyclohexanone.
La fraction de queue Q1 est introduite dans une seconde colonne de distillation 6 comprenant également 22 plateaux théoriques.
La fraction F2 récupérée en tête est de la cyclohexanone à un degré de pureté supérieur à 99,8 % et présente au test UV à une longueur d'onde de 230 nm, une transmission de 88,5 %.

Dans le mode de réalisation illustré, la fraction de queue Q2 peut être alimentée dans une troisième colonne à distiller 7 permettant de séparer les produits lourds (produits de point d'ébullition plus élevé que celui de la cyclohexanone) sous forme d'une fraction Q3. La fraction de tête F3 contenant de la cyclohexanone et du cyclohexanol peut être recyclée dans le réacteur 2 de déshydrogénation.
Selon le mode de réalisation illustré dans la figure annexée la fraction de tête F1 recueillie à partir de la colonne à distiller 5 peut être alimentée dans un décanteur 8 pour séparer la phase aqueuse puis dans une colonne à distiller 9. la fraction de queue Q4 recueillie contenant du cyclohexanone peut être recyclée dans le réacteur 2.
La fraction de tête F4 contenant des produits légers, c'est-à-dire de faible point d'ébullition, est traitée comme un effluent.

### Exemple 3:

L'exemple 2 a été répété mais en alimentant dans le réacteur un mélange contenant 59% en poids de cyclohexanone, 39 % en poids de cyclohexanol 0,5% d'eau et 1,5 d'impuretés lourdes ou légères dont 360 ppm de cyclopenténal.

Le débit d'alimentation de ce mélange dans le réacteur 2 est de 135 g/h.
La température du réacteur est de 270°C..
La composition du milieu réactionnel en sortie du réacteur 2 est : 75,2 % en poids de cyclohexanone, 22,3 % en poids de cyclohexanol et des impuretés lourdes ou légères. La concentration en cyclopenténal dans ce milieu est inférieure au seuil de détection, c'est à dire inférieure à 30 ppm. Le taux de transformation du cyclohexanol en cyclohexanone est de 44%:
La cyclohexanone récupéré comme fraction F2 présente une teneur en cyclopenténal inférieure 30mg/kg et une transmission dans le test UV à 230nm de 89,5%.

## Revendications

1. Procédé de fabrication de cyclohexanone **caractérisé en ce qu'**il comprend les étapes suivantes :
• Oxyder du cyclohexane en hydroperoxyde de cyclohexyle par de l'oxygène en absence de catalyseur
• Purifier le milieu réactionnel par lavage à l'eau
• Décomposer l'hydroperoxyde de cyclohexyle en cyclohexanol et cyclohexanone en présence d'un catalyseur.
• Récupérer le mélange cyclohexanol/cyclohexanone par séparation du cyclohexane n'ayant pas réagi et séparation des produits de point d'ébullition plus élevés que ceux du cyclohexanol/cyclohexanone
• Déshydrogèner le cyclohexanol contenu dans le mélange cyclohexanol/cyclohexanone en présence d'un catalyseur de déshydrogénation
• Distiller, dans une première étape de distillation, le mélange obtenu pour obtenir une fraction de tête (F₁) comprenant les composés de point d'ébullition inférieur à la température d'ébullition de la cyclohexanone, et une fraction de queue (Q₁)
• Distiller, dans une deuxième étape de distillation, la fraction de queue (Q₁) pour obtenir une fraction de tête (F₂) constitué par la cyclohexanone, et une fraction de queue (Q₂)

2. Procédé selon la revendication 1, **caractérisé en ce que** la fraction de queue Q₂ est distillée, dans une troisième étape de distillation, pour obtenir une fraction de tête (F₃) constituée de cyclohexanol/cyclohexanone et une fraction de queue (Q₃) constitué par des composés de point d'ébullition élevés.

3. Procédé selon la revendication 2, **caractérisé en ce que** la fraction de tête (F₃), est mélangée au mélange cyclohexanol/cyclohexanone introduit dans l'étape de déshydrogénation.

4. Procédé selon l'une des revendications précédentes, **caractérisé en ce que** la fraction de tête (F₁) est distillée, dans une quatrième étape de distillation, pour obtenir une fraction de tête (F₄) constituée par des composés de point d'ébullition bas, et une fraction de queue (Q₄) constituée de cyclohexanone.

5. Procédé selon la revendication 4, **caractérisé en ce que** la fraction de queue (Q₄) est ajouté au mélange cyclohexanol/cyclohexanone introduit à l'étape de déshydrogénation.

6. Procédé selon l'une des revendications précédentes, **caractérisé en ce que** l'étape de déshydrogénation est mise en oeuvre en présence d'un catalyseur choisi dans le groupe comprenant les oxydes de cuivre, de magnésium, de zinc et leurs mélanges.

7. Procédé selon l'une des revendications précédentes, **caractérisé en ce que** la cyclohexanone obtenu en fraction de tête (F2) est utilisé comme matière première pour la fabrication de ε-caprolactame.

## Claims

1. Method for producing cyclohexanone **characterized in that** it comprises the following steps:
• oxidation of cyclohexane to cyclohexyl hydroperoxide by oxygen in the absence of catalyst
• purification of the reaction mixture by washing with water
• decomposition of the cyclohexyl hydroperoxide to cyclohexanol and cyclohexanone in the presence of a catalyst
• recovery of the cyclohexanol/cyclohexanone mixture by the separation of unreacted cyclohexane and the separation of products with higher boiling points than cyclohexanol/cyclohexanone
• dehydrogenation of the cyclohexanol present in the cyclohexanol/cyclohexanone mixture in the presence of a dehydrogenation catalyst
• distillation of the mixture obtained in a first distillation step in order to obtain a top fraction (F₁) comprising the compounds with lower boiling points than cyclohexanone, and a bottom fraction (Q₁)
• distillation of the bottom fraction (Q₁) in a second distillation step to obtain a top fraction (F₂) consisting of cyclohexanone, and a bottom fraction (Q₂)

2. Method according to Claim 1, **characterized in that** the bottom fraction Q₂ is distilled in a third distillation step to obtain a top fraction (F₃) consisting of cyclohexanol/cyclohexanone and a bottom fraction (Q₃) consisting of high boiling point compounds.

3. Method according to Claim 2, **characterized in that** the top fraction (F₃) is mixed with the cyclohexanol/cyclohexanone mixture introduced in the dehydrogenation step.

4. Method according to one of the preceding claims, **characterized in that** the top fraction (F₁) is distilled, in a fourth distillation step, in order to obtain a top fraction (F₄) consisting of low boiling point compounds, and a bottom fraction (Q₄) consisting of cyclohexanone.

5. Method according to Claim 4, **characterized in that** the bottom fraction (Q₄) is added to the cyclohexanol/cyclohexanone mixture introduced in the dehydrogenation step.

6. Method according to one of the preceding claims, **characterized in that** the dehydrogenation step is carried out in the presence of a catalyst selected from the group comprising copper, magnesium and zinc oxides and mixtures thereof.

7. Method according to one of the preceding claims, **characterized in that** the cyclohexanone obtained in the top fraction (F₂) is used as a raw material for producing ε-caprolactam.

## Patentansprüche

1. Verfahren zur Herstellung von Cyclohexanon, **dadurch gekennzeichnet, daß** man:
• Cyclohexan in Abwesenheit von Katalysator mit Sauerstoff zu Cyclohexylhydroperoxid oxidiert,
• die Reaktionsmischung durch Waschen mit Wasser reinigt,
• das Cyclohexylhydroperoxid in Anwesenheit eines Katalysators zu Cyclohexanol und Cyclohexanon zersetzt,
• das Cyclohexanol/Cyclohexanon-Gemisch durch Abtrennung des nicht umgesetzten Cyclohexans und Abtrennung von Produkten mit höherem Siedepunkt als Cyclohexanol/Cyclohexanon gewinnt,
• das in dem Cyclohexanol/Cyclohexanon-Gemisch enthaltene Cyclohexanol in Anwesenheit eines Dehydrierungskatalysators dehydriert,
• in einem ersten Destillationsschritt das erhaltene Gemisch destilliert, wobei man eine Kopffraktion (F₁), die die Verbindungen mit niedrigerem Siedepunkt als Cyclohexanon enthält, und eine Sumpffraktion (Q₁) erhält,
• in einem zweiten Destillationsschritt die Sumpffraktion (Q₁) destilliert, wobei man eine Kopffraktion (F₂), die aus Cyclohexanon besteht, und eine Sumpffraktion (Q₂) erhält.

2. Verfahren nach Anspruch 1, **dadurch gekennzeichnet, daß** man in einem dritten Destillationsschritt die Sumpffraktion Q₂ destilliert, wobei man eine Kopffraktion (F₃), die aus Cyclohexanol/Cyclohexanon besteht, und eine Sumpffraktion (Q₃), die aus Verbindungen mit hohem Siedepunkt besteht, erhält.

3. Verfahren nach Anspruch 2, **dadurch gekennzeichnet, daß** man die Kopffraktion (F₃) mit dem in den Dehydrierungsschritt eingetragenen Cyclohexanol/Cyclohexanon-Gemisch mischt.

4. Verfahren nach einem der vorhergehenden Ansprüche, **dadurch gekennzeichnet, daß** man in einem vierten Destillationsschritt die Kopffraktion (F₁) destilliert, wobei man eine Kopffraktion (F₄), die aus Verbindungen mit niedrigem Siedepunkt besteht, und eine Sumpffraktion (Q₄), die aus Cyclohexanon besteht, erhält.

5. Verfahren nach Anspruch 4, **dadurch gekennzeichnet, daß** man die Sumpffraktion (Q₄) dem in den Dehydrierungsschritt eingetragenen Cyclohexanol/Cyclohexanon-Gemisch zusetzt.

6. Verfahren nach einem der vorhergehenden Ansprüche, **dadurch gekennzeichnet, daß** man den Dehydrierungsschritt in Gegenwart eines Katalysators aus der Gruppe bestehend aus Kupfer-, Magnesium- und Zinkoxiden und Mischungen davon durchführt.

7. Verfahren nach einem der vorhergehenden Ansprüche, **dadurch gekennzeichnet, daß** man das in der Kopffraktion (F₂) erhaltene Cyclohexanon als Ausgangsstoff zur Herstellung von ε-Caprolactam verwendet.
